# EUROPÄISCHE PATENTANMELDUNG

(11) **EP 1 715 302 A1**
(43) Veröffentlichungstag der Anmeldung: **25.10.2006**
(21) Anmeldenummer: 06007264.2
(22) Anmeldetag: 06.04.2006
(51) Int. Cl.: G01F 1/74, G01F 1/05, G01F 1/68, G01F 1/66, A61L 2/06, G01N 25/60, G01N 7/00

(54) **Verfahren und Vorrichtung zur Messung von nichtkondensierbaren Gasen und Dämpfen in einem Dampf-Gasgemisch**

(30) Priorität: 21.04.2005 DE 102005018707
(71) Anmelder: SIMICON Gesellschaft für Hygiene-, Umwelt- und Sicherheitstechnik mbH, 81829 München (DE)
(72) Erfinder: Bönisch, Michael, 80339 München (DE)
(74) Vertreter: von Bülow, Tam

(57) **Zusammenfassung**

Das Verfahren und die Vorrichtung zur Messung von nichtkondensierbaren Gasen und Dämpfen in einem Dampf-Gasgemisch leitet kontinuierlich oder nichtkontinuierlich eine Probenmenge des Dampf-Gasgemisches durch einen Kondensator (2), aus dem ein Gemisch aus Kondensat (3) und Gasblasen (4) und eine Leitung (5) austritt. An die Leitung (5) ist ein Sensor (6) angeschlossen, der einen Mengenstrom nur einer der Komponenten des Gemisches aus Kondensat (3) und Gasblasen (4) quantitativ mißt und ein entsprechendes Ausgangssignal an eine Auswerteeinheit (9) liefert. Diese überwacht zusätzlich signifikante Änderungen des Ausgangssignals des Sensors (6) und ermittelt daraus rechnerisch den Mengenstrom der anderen Komponente. Der Mengenstrom kann dabei das Volumen pro Zeiteinheit oder die Masse pro Zeiteinheit der Komponente sein. Die Komponente, die von dem Sensor (6) gemessen wird, kann alternativ entweder das Kondensat oder das Gas sein.

## Beschreibung

Die vorliegende Erfindung betrifft ein Verfahren und eine Vorrichtung zur Messung von nichtkondensierbaren Gasen und Dämpfen in einem Dampf-Gasgemisch gemäß dem Oberbegriff des Patentanspruches 1 bzw. 7.

Medizinische Produkte werden vorwiegend in Dampfsterilisatoren mit Sattdampf höchster Güte sterilisiert. Die Verwendung von Sattdampf hoher Reinheit kommt ebenso bei der Desinfektion von Desinfektionsgut oder Lebensmitteln zur Anwendung. Sattdampf wird vorwiegend im Dampfgenerator aus vollentsalztem Wasser hergestellt. Dieses Wasser enthält einen mehr oder weniger hohen Anteil an nichtkondensierbaren Gasen.

Die nichtkondensierbaren Gase werden bei der Einspeisung in den Dampfgenerator spontan freigesetzt, da sich deren Löslichkeitsprodukt ab einer Temperatur von ca. 105°C stark absenkt. Der Sattdampf wird bei der Entnahme mit den freigesetzten nichtkondensierbaren Gasen in den Sterilisator oder die Desinfektionsanlage eingebracht. Der Sattdampf gibt dort die für die erforderliche Abtötung der Mikroorganismen nötige hohe Energie ab. Befinden sich jedoch Anteile von nichtkondensierbaren Gasen im Sattdampf, so verhindern diese die Sterilisier- oder Desinfizierwirksamkeit des Sattdampfes durch Bildung von sog. Luftinseln. Nicht nur das Vorhandensein von nichtkondensierbaren Gasen im Dampf führt zur Unsterilität am Medizinprodukt, sondern auch bei Leckagen im Rohrleitungssystem können trotz Überdruck, bedingt durch den Venturieffekt, beträchtliche Anteile an nichtkondensierbaren Gasen, in diesem Falle Luft, in den Dampf gelangen. Ebenso sind Leckagen im Sterilisations- oder Desinfektionsgerät verantwortlich für beträchtliche Anteile an nichtkondensierbaren Gasen im Behandlungsgut. Programmfehler in der Steuerung können bei den vakuumfraktionierten Verfahren zu erhöhten Restluftmengen führen, die sich wie die nichtkondensierbaren Gase Verhalten und ebenso zu Luftinseln führen. Diese nichtkondensierbaren Gase bestehen u.a. aus Stickstoff, Sauerstoff, Kohlendioxid und Edelgasen.

Zur Sicherstellung einer einwandfreien Sterilisation oder Desinfektion ist die Reduzierung dieser Gase im Sattdampf erforderlich, wofür eine kontinuierliche und exakte Messung und Überwachung der Anteile der nichtkondensierbaren Gase im Dampf als auch in der Sterilisations- oder Desinfektionskammer unabdingbar ist.

Zur Messung des Anteiles von nichtkondensierbaren Gasen und Dämpfen in einem Dampf-Gasgemisch sind folgende Verfahren und Vorrichtungen bekannt:

Die DE 28 46 826 A1 schlägt vor, einen Anteil des Dampfes durch einen Kühler zu leiten, wo sich Kondensat (Flüssigkeit) und Gasblasen bilden, die mittels einer Analyseleitung in eine Probenahmestation geleitet werden. Die Probenahmestation weist ein vertikal stehendes Meßrohr auf, in welchem die Gasblasen in der Flüssigkeit nach oben steigen, so daß nach einer Wartezeit Gas und Flüssigkeit getrennt sind und der Flüssigkeitspegel an dem Meßrohr abgelesen werden kann. Die im Meßrohr herrschenden Druck- und Temperaturwerte werden gemessen und zur Umrechnung auf Normbedingungen verwendet. Diese Vorrichtung arbeitet somit diskontinuierlich.

Die DE 36 36 716 A1 schlägt ebenfalls vor, den Dampf zu kondensieren und das Gas/Kondensatgemisch durch eine Kapillareinheit zu leiten, in welchem sich aufeinanderfolgend Kondensatabschnitte und Gasabschnitte aneinanderreihen, die mit einer Meßeinrichtung untersucht werden. Die Meßeinrichtung kann eine Lichtschranke, eine kapazitive oder eine Widerstandsmeßeinrichtung sein. Die Meßeinrichtung kann lediglich zwischen Kondensat und Gas unterscheiden, so daß Voraussetzung für ein quantitativ brauchbares Meßergebnis ist, daß das Gemisch mit sehr konstanter Strömungsgeschwindigkeit fließt. Änderungen der Strömungsgeschwindigkeit haben große Meßfehler zur Folge. Durch den Vorgang der Kondensation des Dampfes im Kondensator ergeben sich aber Schwankungen der Strömungsgeschwindigkeit durch die enorme Volumenreduzierung bei der Kondensation des Dampfes im Kondensator, wohingegen die nichtkondensierbaren Gase nur unwesentlich an Volumen abnehmen. Kommen größere Anteile an nichtkondensierbaren Gasen in den Kondensator, so werden die entstehenden Gasblasen durch den nachströmenden Dampf mit hoher Geschwindigkeit durchgeschoben. Bildet sich hinter diesen Gasblasen neues Kondensat, so gerät die Strömung des Kondensates und der Gasblasen zum Stocken oder diese bleiben ganz stehen, da sich jetzt wieder das Dampfvolumen stark reduziert, bis sich nach einiger Zeit der normale Zustand eingependelt hat. Die Bildung dieser Kondensat- und Gasblasen führt zu einer nicht kontrollierbaren Strömungsgeschwindigkeit und somit zu einer großen Ungenauigkeit der Messung. Eine Beruhigungsstrecke ist für diesen Fall wegen der steigenden Kohäsions- und Fließkräfte nicht ausreichend realisierbar, zumal eine zu lange Beruhigungsstrecke wegen des Löslichkeitsproduktes von Gasen zur Rückbildung und somit zur Verfälschung des Meßergebnisses führt. Temperaturschwankungen führen durch die Volumenänderung der Gasblasen ebenfalls zur Verfälschung des Meßergebnisses. Eine Justierung ist aus diesen Gründen nur begrenzt durchführbar. Bei Messung mit einer Lichtschranke kann darüber hinaus die Verwendung einer Glaskapillare die Meßfunktion durch Verschmutzungen unwirksam machen.

Die DE 43 19 402 A1 schlägt vor, ein Dampf-Gasgemisch nach Leiten durch einen Kühler in eine Meßeinrichtung zu leiten, die ein Steigrohr enthält. Durch Auswiegen der sich in dem Steigrohr bildenden Flüssigkeitssäule kann der Anteil des nichtkondensierbaren Gases bestimmt werden. Problematisch dabei ist die Befüllung des Steigrohres. Da die Messung im Prinzip über ein Zeitintegrall erfolgt, müßte auch hier eine möglichst konstante Strömungsgeschwindigkeit vorliegen. Durch den statischen Druck in einem Steigrohr sind die Gasblasen am unteren Ende kleiner als im oberen Ende, was bedingt durch die Unbestimmbarkeit des Auftretens einer Gasblase zu einem zufälligen Meßfehler führt. Da der stokes'sche Auftrieb nur schwach wirksam wird, erfordert diese Vorrichtung eine größere Probenahme, was zu größerem Energieverlust und einem erhöhten Wasserverbrauch zur Kondensation der Probe führt.

Die DE 196 46 301 C1 leitet nach Kondensation das Gas/Kondensat-Gemisch in einen Meßbehälter, wo die Gasblasen sich wiederum von dem Kondensat trennen und die Größe der resultierenden Gasblase optoelektronisch gemessen wird.

Die DE 102 39 609 A1 arbeitet in ähnlicher Weise mit einem Gasseparator, dem nach Kondensation das Kondensat-Gas-Gemisch zugeführt wird und die Kondensat- und Gasanteile getrennt über Druck- und Füllstandsmessungen einer Kondensatsäule quantitativ bestimmt und zur Anzeige gebracht werden. Auch hier muß diskontinuierlich eine bestimmte Probenmenge gezogen und analysiert werden. Eine Justierung ist abhängig von der Art der Zusammensetzung an Kondensat und nichtkondensierbaren Gasen und deshalb nur begrenzt möglich.

Aufgabe der Erfindung ist es, Verfahren und Vorrichtung der eingangs genannten Art dahingehend weiterzubilden, daß mit hoher Genauigkeit die Anteile an Kondensat und nichtkondensierbaren Gasen quantitativ bestimmt werden können. Stark schwankende Strömungsgeschwindigkeiten des Kondensates und der Gasblasen sollen das Meßergebnis nicht verfälschen. Eine Justierung des Meßergebnisses soll leicht möglich sein. Zusätzlich soll ein im wesentlichen kontinuierliches Meßsignal geliefert werden, daß den zeitlichen Verlauf der Anteile an nichtkondensierbaren Gasen darstellt. Weiter sollen nur kleine Probenvolumina entnommen werden müssen, damit die Verlustwärme und/oder die benötigten Kühlenergien reduziert werden. Schließlich soll die Messung auch schmutzunempfindlich sein.

Diese Aufgabe wird durch die im Patentanspruch 1 bzw. 7 angegebenen Merkmale gelöst. Vorteilhafte Ausgestaltungen und Weiterbildungen der Erfindung sind den Unteransprüchen zu entnehmen.

Das Grundprinzip der Erfindung liegt darin, das Gas/Kondensat-Gemisch durch einen Durchflußmesser zu leiten, der quantitativ den Mengenstrom nur einer der Komponenten, also des Kondensats (Flüssigkeit) oder des nichtkondensierbaren Gases, mißt. Der Mengenstrom sei dabei definiert als Volumen pro Zeiteinheit oder als Masse pro Zeiteinheit.

Als Durchflußmesser können alle bekannten Meßgeräte für den Durchfluß von Flüssigkeiten oder Gasen verwendet werden. Es eignen sich also beispielsweise mechanische Durchflußmesser mit Flügelrad, thermische Durchflußmesser, wie sie beispielsweise in der WO 01/84087 A1 beschrieben sind, Durchflußmesser nach dem Ultraschallprinzip, nach dem Laser-Dopplereffekt, Durchflußmesser mit Anemometer oder mit Thermoelementen.

Der Erfindung liegt nämlich die Erkenntnis zugrunde, daß Durchflußmesser, die auf eines der Medien Gas oder Flüssigkeit ansprechen, beim Auftreffen des anderen Mediums, also Flüssigkeit oder Gas, einen signifikanten Sprung ihres Ausgangssignales aufweisen, der je nach Art des angewandten Meßprinzips und zu messenden Mediums eine positive, d.h. ansteigende, oder negative, d.h. abfallende, Flanke hat. Beispielsweise wird ein mechanisch arbeitender Durchflußmesser nach dem Flügelradprinzip bei Anströmung durch eine Flüssigkeit deren Volumen exakt messen. Sobald eine Gasblase an ihm vorbeiströmt, wird er aufgrund der wesentlich geringeren Dichte des Gases einen signifikanten Abfall seiner Drehzahl signalisieren und, nachdem die Gasblase vorbeigeströmt ist, bei wiederum ankommendem Kondensat einen signifikanten Anstieg seiner Drehzahl signalisieren. Da man in der Praxis davon ausgehen kann, daß der Anteil der Gasblasen im Kondensat relativ gering ist, wird die Strömungsgeschwindigkeit des Kondensates vor und hinter der Gasblase in Strömungsrichtung im wesentlichen konstant sein, so daß man davon ausgehen kann, daß während des vergleichsweise kurzen Vorbeiströmens der Gasblase an dem Sensor keine Geschwindigkeitsänderungen auftreten. Somit kann aus dem Zeitintervall zwischen der abfallenden Flanke zu Beginn der Gasblase und der ansteigenden Flanke am Ende der Gasblase auch deren Volumen ermittelt werden. Damit liegt im Beispiel des Durchflußmessers für Flüssigkeiten einerseits ein exakter Meßwert für das Volumen oder die Masse der Flüssigkeit vor und zusätzlich läßt sich das Volumen (oder die Masse) des nichtkondensierbaren Gases bestimmen. Die Messung kann kontinuierlich vorgenommen werden und liefert kontinuierlich Ausgangssignale für die Zusammensetzung des Gas/Kondensat-Gemisches.

Da die Gasblasen in unregelmäßigen zeitlichen Abständen und in unregelmäßiger Größe auftreten, hängt die Genauigkeit des Meßergebnisses von der Meßdauer ab und wird mit zunehmender Meßdauer genauer. Zweckmäßigerweise werden die Meßergebnisse über eine vorgegebene Meßdauer aufintegriert bzw. aufsummiert und dann ein zeitlicher Mittelwert gebildet. Diese Meßdauer liegt beispielsweise bei einigen Minuten bis ca. 5 Minuten.

Die Auswertung kann auch in der Weise erfolgen, daß jeweils die Meßwerte einer vorgegebenen Zeitdauer ausgewertet werden, wobei aktuelle, neue Meßwerte hinzugefügt und ältere Meßwerte eliminiert werden.

Die Erfindung ist somit auch bei stark schwankenden Strömungsgeschwindigkeiten einer zu untersuchenden Probe anwendbar und liefert ein genaues Ergebnis der Volumen- oder Massenströme des Kondensates und des Gases, aus deren Verhältnis sich der Anteil der nichtkondensierbaren Gase im Dampf reproduzierbar ermitteln läßt. Eine Justierung der Ausgangssignale zur Kalibrierung gegenüber einer Referenz und die Darstellung des zeitlichen Verlaufes zur Überwachung der Sterilisier- oder Desinfizierwirksamkeit während Sterilisier- oder Desinfizierphasen ist somit ein wichtiger Vorteil der Erfindung. Die volle Funktionsfähigkeit ist auch bei Ablagerungen von Kondensatrückständen und geringen Massen oder Volumina der entnommenen Probe möglich, womit die Verlustleistungen für Abwärme oder Abwasser gesenkt werden können.

In Kombination mit einer Vorrichtung gemäß DE 199 29 588 A1 kann mit der Erfindung auch der Dampfgehalt X und die Überhitzung des Hauptdampfstromes exakt ermittelt werden, was mit der bekannten Vorrichtung alleine nicht möglich ist.

Im folgenden wird die Erfindung anhand eines Ausführungsbeispieles im Zusammenhang mit der Zeichnung ausführlicher erläutert. Es zeigt:
- Fig. 1: eine schematisch stark vereinfachte Skizze einer Vorrichtung nach der Erfindung; und
- Fig. 2: einen schematischen zeitlichen Volumen- oder Massenstrom des verwendeten Durchflußsensors.

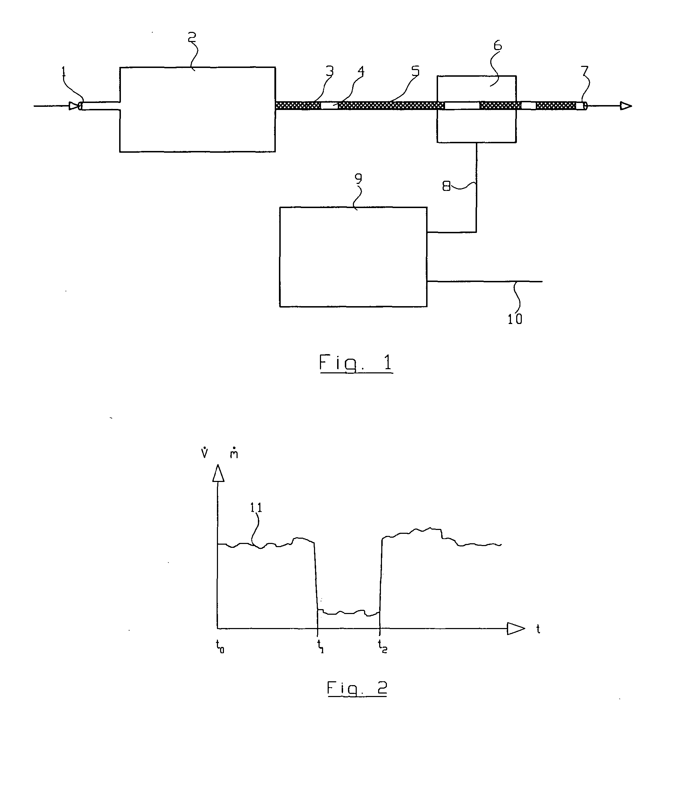

In Fig. 1 ist eine Dampfleitung 1 dargestellt, durch die eine Probenmenge eines Dampf-Gasgemisches in einen Kondensator 2 geleitet wird. Das Dampf-Gasgemisch stammt beispielsweise aus einer nicht dargestellten Sterilisier- oder Desinfizierkammer. In der Dampfleitung 1 kann ein nicht dargestelltes Absperrventil angeordnet sein, um eine Probenahme einzuleiten bzw. zu beenden, wobei noch darauf hinzuweisen ist, daß bezogen auf die gesamte Dampfmenge in der Sterilisier- oder Desinfizierkammer nur ein geringes Volumen entnommen werden muß. In dem Kondensator 2 wird das Dampf-Gasgemisch so weit abgekühlt, daß der Dampf zu Flüssigkeit kondensiert, so daß sich im Kondensator ein Flüssigkeits-Gas-Gemisch bildet. Dieses Gemisch aus Kondensat 3 und Gasblasen 4 wird über eine Leitung 5 einem Durchflußsensor 6 zugeführt, aus dem es über einen Austritt 7 ausfließen kann. Am Austritt 7 herrscht Atmosphärendruck. Der Durchflußsensor 6 liefert ein Ausgangssignal 11 (Fig. 2) über eine elektrische Leitung 8 an eine Recheneinheit, in der eine Signalauswertung erfolgt und über eine Ausgangsleitung 10 ein Ausgangssignal 11 ausgegeben wird.

Der Durchflußmesser 6 mißt das Volumen oder die Masse einer der beiden Komponenten des Gemisches aus Kondensat 3 und Gasblasen 4. Als Sensor kann jeder bekannte Sensor für das Volumen oder die Masse einer Flüssigkeit oder eines Gases verwendet werden. Wichtig ist, daß der Sensor das Volumen oder die Masse dieser einen Komponente quantitativ mißt und ein entsprechendes Ausgangssignal, das dieser gemessenen Menge entspricht, als Signal 11 ausgibt.

Als mögliche Sensoren kommen u.a. in Betracht: thermische Durchflußsensoren, anemometrische Durchflußsensoren, Durchflußsensoren mit Thermoelementen, mit Flügelrad, nach dem Laser-Dopplereffekt arbeitende Sensoren, Ultraschallsensoren. Es sei betont, daß diese Aufzählung unvollständig ist und jegliche Art von Durchflußsensoren für die Bestimmung des Volumens oder der Masse einer Flüssigkeit oder eines Gases verwendet werden können. Da keine Sensoren existieren, die beide Komponenten, also Flüssigkeit und Gas, quantitativ exakt messen können, genügt es nach der Erfindung, einen Sensors zu verwenden, der eine von beiden Komponenten, also Gas oder Flüssigkeit, exakt mißt.

Zur weiteren Erläuterung wird nun auf Fig. 2 Bezug genommen, die ein Ausgangssignal 11 des Sensors 6 zeigt. Dort ist der Volumenstrom dV/dt (Volumen pro Zeiteinheit) oder der Massenstrom dm/dt (Masse pro Zeiteinheit) über der Zeitachse t abgebildet. Es sei angenommen, daß der Sensor 6 des Typs ist, der den entsprechenden Strom einer Flüssigkeit mißt. Im Zeitraum t₀ bis t₁ sei angenommen, daß Kondensat 3 durch den Sensor 6 strömt. Das Ausgangssignal 11 ist demnach proportional dem Volumen- oder Massenstrom der Flüssigkeit. Zum Zeitpunkt t₁ komme nun eine Gasblase 4 zum Sensor 6. Das Ausgangssignal 11 weist dann einen signifikanten Abfall auf, der das Vorhandensein einer Gasblase eindeutig identifizieren läßt. Im Zeitraum t₁ bis t₂ fließt die Gasblase durch den Sensor. Das Ausganssignal ist in diesem Zeitraum deutlich niedriger als im Zeitraum t₀ bis t₁. Seine Größe ist nicht weiter zu beachten, da sie in Bezug auf den Volumen- oder Massenstrom der Gasblase keine Relevanz hat und als Meßwert somit nicht zu gebrauchen ist. Zum Zeitpunkt t₂ ist der Durchfluß der Gasblase 4 beendet und es strömt wieder Kondensat 3 durch den Sensor 6, was durch einen signifikanten Anstieg des Ausgangssignales des Sensors 6 erkannt wird, wobei ab dem Zeitpunkt t₂ das Ausgangssignal des Sensors wiederum ein genauer Meßwert des Volumen- oder Massenstromes des Kondensates ist. Die Zeitdauer t₁ bis t₂ wird von der Recheneinheit 9 ausgewertet und läßt Rückschlüsse auf das Volumen oder die Masse der Gasblase zu, da diese mit derselben Geschwindigkeit durch die Leitung 5 und den Sensor 6 fließt, wie das Kondensat. Da das Volumen der Gasblase im Verhältnis zum Volumen des benachbarten Kondensates klein ist, werden während des Durchströmens der Gasblase durch den Sensor 6 keine wesentlichen Änderungen der Strömungsgeschwindigkeit durch die Leitung 5 und den Sensor 6 auftreten. Bei bekanntem Querschnitt der Leitung 5 läßt sich somit aus der Zeitdauer t₁ bis t₂, also der Zeitdauer zwischen abfallender Flanke und ansteigender Flanke des Ausgangssignals des Sensors das Volumen der Gasblase ermitteln. Aus dem Verhältnis des Volumens der Gasblasen zum Volumen des Kondensates läßt sich somit der Anteil der nichtkondensierbaren Gase im Dampf ermitteln und bedarfsweise mit 100 multipliziert in Prozent ausgeben.

Wird als Sensor ein Sensor für den Volumenstrom verwendet, so ergibt sich der Massenstrom des untersuchten Kondensates aus dessen Dichte multipliziert mit dessen Volumenstrom.

## Patentansprüche

1. Verfahren zur Messung von nichtkondensierbaren Gasen und Dämpfen in einem Dampf-Gasgemisch, bei dem eine Probenmenge des Dampf-Gasgemisches kondensiert wird und das daraus resultierende Gemisch aus Kondensat und Gasblasen einem Sensor zugeführt wird,
**dadurch gekennzeichnet,**
**daß** der Sensor einen Mengenstrom nur einer der Komponenten des Gemisches aus Kondensat und Gasblasen quantitativ mißt und
**daß** eine Auswerteeinheit aus signifikanten Änderungen eines Ausgangssignales des Sensors den Mengenstrom der anderen Komponente des Gemisches aus Kondensat und Gasblasen ermittelt.

2. Verfahren nach Anspruch 1, **dadurch gekennzeichnet, daß** der Mengenstrom jeweils das durch den Sensor fließende Volumen pro Zeiteinheit ist.

3. Verfahren nach Anspruch 1, **dadurch gekennzeichnet, daß** der Mengenstrom die jeweils durch den Sensor fließende Masse pro Zeiteinheit ist.

4. Verfahren nach einem der Ansprüche 1 bis 3, **dadurch gekennzeichnet,**
**daß** der Sensor den Mengenstrom des Kondensates mißt.

5. Verfahren nach einem der Ansprüche 1 bis 3, **dadurch gekennzeichnet,**
**daß** der Sensor den Mengenstrom der Gasblasen mißt.

6. Verfahren nach einem der Ansprüche 1 bis 5, **dadurch gekennzeichnet,**
**daß** die Auswerteeinheit aus dem Verhältnis des Mengenstromes der Gasblasen zum Mengenstrom des Kondensates den Anteil der nichtkondensierbaren Gase im Dampf ermittelt.

7. Vorrichtung zur Messung von nichtkondensierbaren Gasen und Dämpfen in einem Dampf-Gasgemisch mit einem Kondensator (2), dem das Dampf-Gasgemisch zuführbar ist, mit einer an den Kondensator ausgangsseitig angeschlossenen Leitung (5), dem ein aus dem Kondensator (2) stammendes Gemisch aus Kondensat (3) und Gasblasen (4) zuführbar ist, und mit einem an die Leitung (5) angeschlossenen Sensor (6),
**dadurch gekennzeichnet,**
**daß** der Sensor (6) derart ausgebildet ist, daß er nur eine Komponente des Gemisches aus Kondensat (3) und Gasblasen (4) quantitativ mißt, und
**daß** der Ausgang des Sensors (6) mit einer Auswerteeinheit (9) verbunden ist, die aus signifikanten Änderungen des Ausgangssignales (11) des Sensors (9) den Mengenstrom der anderen Komponente ermittelt.

8. Vorrichtung nach Anspruch 7, **dadurch gekennzeichnet, daß** der Sensor (6) so ausgebildet ist, daß er das Volumen pro Zeiteinheit einer der Komponenten mißt.

9. Vorrichtung nach Anspruch 7, **dadurch gekennzeichnet, daß** der Sensor (6) so ausgebildet ist, daß er die durch den Sensor fließende Masse pro Zeiteinheit mißt.

10. Vorrichtung nach einem der Ansprüche 7 bis 9, **dadurch gekennzeichnet,**
**daß** der Sensor (6) als Flüssigkeitssensor ausgebildet ist.

11. Vorrichtung nach einem der Ansprüche 7 bis 9, **dadurch gekennzeichnet,**
**daß** der Sensor (6) als Gassensor ausgebildet ist.

12. Vorrichtung nach einem der Ansprüche 7 bis 11, **dadurch gekennzeichnet,**
**daß** ein Ausgang des Sensors (6), aus dem das Gemisch aus Kondensat (3) und Gasblasen (4) austritt, mit Atmosphärendruck verbunden ist.
